(19) Europäisches Patentamt
European Patent Office
Office européen des brevets

(11) **EP 1 765 307 B1**

(12) **EUROPÄISCHE PATENTSCHRIFT**

(45) Veröffentlichungstag und Bekanntmachung des Hinweises auf die Patenterteilung:
**21.11.2012 Patentblatt 2012/47**

(21) Anmeldenummer: 05735962.2

(22) Anmeldetag: **12.05.2005**

(51) Int Cl.:
*A61K 9/58* *(2006.01)*        *A61K 9/32* *(2006.01)*

(86) Internationale Anmeldenummer:
**PCT/CH2005/000264**

(87) Internationale Veröffentlichungsnummer:
**WO 2005/115352 (08.12.2005 Gazette 2005/49)**

(54) **TROCKENBESCHICHTUNGSVERFAHREN**

DRY-COATING PROCESS

PROCEDE DE REVETEMENT A SEC

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **24.05.2004 CH 886042004**

(43) Veröffentlichungstag der Anmeldung:
**28.03.2007 Patentblatt 2007/13**

(73) Patentinhaber: **Mepha Schweiz AG**
**4147 Aesch (CH)**

(72) Erfinder:
• **ENGELMANN, Stephan**
**CH-4143 Reinach (CH)**

• **BETZING, Jürgen**
**79539 Lörrach (DE)**

(74) Vertreter: **Braun, André jr.**
**Braunpat Braun Eder AG**
**Reussstrasse 22**
**Postfach**
**4015 Basel (CH)**

(56) Entgegenhaltungen:
**EP-A- 0 387 885     US-A- 4 296 230**
**US-A- 4 711 784     US-B1- 6 656 984**

**EP 1 765 307 B1**

## Beschreibung

Die vorliegende Erfindung betrifft ein Trockenbeschichtungsverfahren für feste Substrate, insbesondere für die Herstellung von beschichteten pharmazeutischen Darreichungsformen, beispielsweise zur Herstellung von beschichteten Tabletten und Pellets, wobei ausschliesslich lösungsmittelfreie Beschichtungskomponenten zur Anwendung kommen.

Die Beschichtung, bzw. Befilmung, von unterschiedlichen Substraten ist bekannt. So ist es bekannt, pharmazeutische Arzneiformen, bzw. Darreichungsformen, herzustellen, indem die Substrate, auch Kerne genannt, mit Beschichtungskomponenten überzogen werden, wobei mindestens eine dieser Komponenten in einem organischen oder anorganische Lösungsmittel, vorzugsweise Wasser, gelöst oder dispergiert ist bzw. als Latex vorliegt. Es ist auch bekannt, zuerst zwei "trockene Komponenten", beispielsweise ein Polymer und einen Weichmacher, und anschliessend eine dritte, Lösungsmittel enthaltende, Komponente auf das Substrat aufzubringen, bzw. die lösungsmittelhaltige Komponente in den Weichmacher einzuarbeiten und derart die gleichmässige Beschichtung herzustellen. Ohne Zugabe eines geeigneten Lösungsmittels, und in der notwendigen Menge, erscheint die stabile, kohärente und gleichmässige Filmbildung, wie eine solche beispielsweise für die Bildung eines enterisch und/oder retardierend und/oder schützend wirkenden oder kosmetischen Films nötig ist, nicht möglich.

Die Verwendung von Lösungsmitteln hat zahlreiche Nachteile. So sind die Verfahrenszeiten verlängert, da das Lösungsmittel entfernt werden muss, insbesondere bei wasserbasierten Systemen. Bei der Verwendung von organischen Lösungsmitteln dürfen zusätzlich nur bis zu gesetzlich festgelegten niedrigen Grenzen Restlösungsmittel im Präparat verbleiben. Die Verwendung von organischen Lösungsmittel ist auch aus Sicht des Umweltschutzes fraglich. Oft sind die zu beschichtenden Arzneiformen auch instabil gegenüber dem Lösungsmittel. Auch bei andern Beschichtungsverfahren, wie beispielsweise die an sich bekannte lösungsmittelfreie Beschichtung mittels Hotmelt bei einer Verfahrenstemperatur von über 80°C oder mittels elektrostatischer Verfahren, ist aufgrund thermischer Stressung mit der Instabilität der zu beschichtenden Arzneimittel zu rechnen.

Es wurde nun gefunden, dass die Kombination ausgewählter Polymere (im weiteren auch Beschichtungspolymer genannt) einerseits und ausgewählter Weichmacher andererseits gleichmässige und kohärente Filme auch ohne die Zugabe eines Lösungsmittels bei niedrigen Prozesstemperaturen ergeben und für die Beschichtung bzw. Befilmung von empfindlichen Substanzen, wie pharmazeutische Kerne, und der lösungsmittelfreien Herstellung enterisch und/oder retardierend und/oder schützend und/oder kosmetisch wirkenden Filmen, sehr geeignet sind. Hierzu ist es erfindungsgemäss nötig, für jede Kombination von Beschichtungspolymer und Weichmacher vorgängig zur Beschichtung die optimalen Temperatur- und Zeitparameter zu bestimmen, wie dies hierin im weiteren beschrieben ist. Dies erfindungsgemäss auszuführen, stellt für den Fachmann kein Problem dar.

Das erfindungsgemässe Verfahren ergibt stark verkürzte Prozesszeiten, ein effizienteres und sicheres Befilmen der Kerne, insbesondere bei Arzneistoffen, die gegenüber Lösungsmittel instabil sind. Derart ergeben sich keine organischen Restlösemittel in der Arzneiform und keine Emissionen umweltschädlicher Lösungsmittel.

Die vorliegende Erfindung ist in den Patentansprüchen definiert. Die vorliegende Erfindung betrifft ein Trockenbeschichtungsverfahren für feste Substrate, insbesondere für die Herstellung von beschichteten pharmazeutischen Darreichungsformen bzw. Arzneiformen, vorzugsweise zur Herstellung von beschichteten Tabletten, Mikrotabletten, feste Lösungen, Kapseln und Pellets, dadurch gekennzeichnet, dass

(i) ausschliesslich lösungsmittelfreie Beschichtungskomponenten zur Anwendung kommen, welche aus (a) mindestens einem Beschichtungspolymer und (b) mindestens einem bei der Beschichtungstemperatur flüssigen Weichmacher bestehen;

(ii) die beiden Komponenten (a) und (b) separat auf die Kerne in dem vorgängig bestimmten Mengenverhältnis und der vorgängig bestimmten Schichtdicke aufgebracht werden und bei der für die kohärente Filmbildung vorgängig bestimmten Temperatur und Zeitdauer so lange gehalten werden, bis sich ein kohärenter Film gebildet hat; wobei

(iii) das Beschichtungspolymer ausgewählt ist aus der Gruppe enthaltend Cellulosederivate, Polyacrylate und Polyvinylderivate, und

(iv) der Weichmacher ausgewählt ist aus der Gruppe enthaltend acetylierte Fettsäureglyceride, Citrate, Phtalate, Sebacate, Glycerin und Glycerinderivate, Polyoxyethylen-Polyoxypropylen-Copolymere, Propylengykole und Rizinusöl.

Die Beschichtungskomponenten können gegebenenfalls "weitere Hilfsstoffe" enthalten, welche "feste Hilfsstoffe" oder "flüssige Hilfsstoffe" sein können. Weitere Hilfsstoffe sind beispielsweise Bindemittel, Trennmittel/- Antiklebemittel, Geschmacksstoffe und Farbstoffe. Solche weitere Hilfsstoffe sind an sich bekannt.

Beispiele für Bindemittel sind HPMC, PVP, PVA; Beispiele für Trennmittel/Antiklebemittel sind Talk und Syloid; Beispiele für Geschmacksstoffe sind synthetische oder natürliche Orangen-, Minz-, Zitronenaromen etc.. Beispiele für Farbstoffe sind natürliche bzw. synthetische Farbstoffe, wie Curcurmin, Lactoflavin, Chinolingelb oder Amaranth.

Die festen Hilfsstoffe können neben dem Beschichtungspolymer separat zugesetzt werden oder vorgängig in dieses eingearbeitet (compoundiert) sein. Vorzugsweise sind die weiteren festen Hilfsstoffe in dem Beschichtungspolymer eingearbeitet. Die weiteren flüssigen Hilfsstoffe werden vorzugsweise mit dem Weichmacher gemeinsam, beispielsweise mittels einer Düse, auf die Oberfläche des Substrats bzw. der Kerne, aufgebracht, wobei darauf zu achten ist, dass die Kombination der Weichmacher und der weiteren Hilfsstoffe bei der Verfahrenstemperatur flüssig sind.

Lösungsmittelfrei im Sinne der vorliegenden Erfindung bedeutet, dass höchstens 20 Gew.-% Lösungsmittel, vorzugsweise höchstens 15 Gew.-% Lösungsmittel, vorzugsweise höchstens 10. Gew.-% Lösungsmittel, vorzugsweise höchstens 5 Gew.-% Lösungsmittel, vorzugsweise höchstens 2 Gew.-% Lösungsmittel, und vorzugsweise höchstens 1-1.5 Gew.-% Lösungsmittel, jeweils bezogen auf das Pelletgewicht bzw. Kerngewicht, im Beschichtungsverfahren, vorzugsweise zusammen mit dem Weichmacher, oder separat, vorzugsweise am Ende des Beschichtungsvorganges, zur Beschichtung aufgetragen wird. Vorzugsweise wird den Beschichtungskomponenten kein Lösungsmittel zugesetzt und kein Lösungsmittel anschliessend in die Beschichtung eingetragen. Das Lösungsmittel ist vorzugsweise Wasser. Der natürliche Wassergehalt des (in der Regel kommerziellen) "trockenen" Polymers oder des "trockenen" Weichmachers" gilt nicht als Lösungsmittel im Sinne der vorliegenden Erfindung.

Bevorzugte Cellulosederivate sind Celluloseether und Celluloseester, insbesondere Methylcellulose (MC), Hydroxypropylmethylcellulose (HPMC), Celluloseacetatphathalat (CAP), Hydroxypropylmethylcellulosemethylphthalat (HPMCMP), Ethylcellulose (EC), Hydroxypropylcellulose (HPC), Hydroxypropylmethylcellulose (HPMC), Hydroxyethylmethylcellulose (HEC), Carboxymethylethylcellulose und Salze (CMEC), Carboxymethylcellulose und ihre Salze (CMC), Hydroxyethylcellulose (HEC), Hydroxypropylmethylcellulosephthalat (HPMCP), Hydroxypropylmethylcelluloseacetatsuccinat (HPMCAS).

Bevorzugte Polyacrylate sind Methacrylsäure-Copolymere, Aminoalkylmethacrylate, und Metharylester-Copolymere, wie Poly(ethylacrylat, methylacrylsäure) (Poly-MA-EA), Poly(methylmethacrylat, methacrylsäure) [Poly-(MA-MMA)], Poly[butylmethacrylat, (2-dimethylaminoethyl)methacrylat, methylmethacrylat] [Poly(BMA-DAEM-MMA)], Poly(ethylacrylat, methylmethacrylat, trimethylammonioethylmethacrylatchlorid) [Poly(EA-MMA-TAMCl)] und Poly(ethylacrylat, methylmethacrylat) [Poly(EA-MMA)].

Bevorzugte Polyvinylderivate sind Polyvinylacetat-Copolymere und Polyvinylpyrrolidone, insbesondere Vinylacetat-Vinylpyrrolidon-Copolymer (PVAc), Polyvinylactatphathalat (PVAP) und Polyvinylpyrrolidon (PVP).

Von den Weichmachern, bzw. von den acetylierten Fettsäureglyceriden sind die Produkte Myvacet K-9-45 bevorzugt; von den Citraten sind Acetyltributylcitrat (ATBC), Acetyltriethylcitrat (ATEC), Tributylcitrat (TBC) und Triethylcitrat (TEC) bevorzugt; von den Phthalaten sind Dibutylphathalat (DBP), Diethylphthalat (DEP) und Dimethylphtalat (DMP) bevorzugt; von den Sebacaten sind Diethylsebacat (DES) und Dibutylsebacat (DBS) bevorzugt. Bevorzugt sind auch Glycerin und Glycerintriacetat, sowie Polyethylenglykolpropoxylat.

Bevorzugt verwendet man 1-50 mg/cm$^2$ an Beschichtungspolymer, vorzugsweise 4-25 mg/cm$^2$.

Das Gewichtsverhältnis von Weichmacher:Beschichtungspolymer ist vorzugsweise 1:50 bis 1:1, vorzugsweise 1:40 bis 2:5.

Das Gewichtsverhältnis der Summe aller "weiterer Hilfsstoffe":Beschichtungspolymer ist vorzugsweise 1:50 bis 1:1, vorzugsweise 1:40 bis 2:5.

Das Verhältnis von Antiklebemittel:Beschichtungspolymer ist vorzugsweise 1:10 bis 2:1, vorzugsweise 1:5 bis 3:2, und insbesondere etwa 1:1. Die Antiklebemittel (vorzugsweise in das Beschichtungspolymer bereits eingearbeitet).

Das Gewichtsverhältnis von Bindemittel:Beschichtungspolymer ist vorzugsweise 1:50 bis 1:1, vorzugsweise 1:40 bis 2:5.

Das Gewichtsverhältnis von Farbstoffe:Beschichtungspolymer ist vorzugsweise 1:100 bis 1:10.

Das Gewichtsverhältnis von Surfactant:Beschichtungspolymer ist vorzugsweise 1:200 bis 1:5.

Das Gewichtsverhältnis von Gleitmittel:Beschichtungspolymer ist vorzugsweise 1:150 bis 4:5.

Das Gewichtsverhältnis von Geschmacksstoffe:Beschichtungspolymer ist vorzugsweise 1:200 bis 1:10.

Die erfindungsgemässe Herstellung von beschichteten pharmazeutischen Darreichungsformen bzw. Arzneiformen, insbesondere zur Herstellung von beschichteten Tabletten, Mikrotabletten, feste Lösungen, Kapseln und Pellets, eignet sich auch für die Herstellung von enterisch und/- oder retardierend und/oder schützend und/oder kosmetisch wirkenden Beschichtungen bzw. beschichteten Darreichungsformen.

Die Beschichtungstemperatur für das Aufbringen der Beschichtungskomponenten (a) und (b), d.i. die Temperatur dieser Beschichtungskomponenten beim Aufbringen auf die Kerne, liegt im Bereich von etwa 45-70°C, vorzugsweise im Bereich von 50-65°C, vorzugsweise bei 50-60°C. Diese Temperatur entspricht der Zulufttemperatur.

Die Kernbetttemperatur, d.h. die Temperatur der zu beschichtenden Kerne in der Wirbelschicht, liegt vorzugsweise im Bereich von 20-55°C, vorzugsweise im Bereich von 40-55°C und insbesondere im Bereich von 45-50°C.

Vorzugsweise wird die Beschichtung nach dem Auftrag der gesamten Auftragsmenge bei der Auftragstemperatur während etwa 30 Minuten bis 120 Minuten, vorzugsweise etwa 60 Minuten bis 90 Minuten belassen (Curing-Zeit), so dass sich ein kohärenter, rissfreier und in der Regel durchscheinender Film bilden kann.

<u>Das Freisetzungsprofil</u>: Mit den derzeit kommerziell verfügbaren beschichteten pharmazeutischen Darreichungsformen wird ein bestimmtes optimales Freisetzungsprofil erreicht. Ein solches Freisetzungsprofil wird beispielsweise mit einem wässerigen Verfahren mit handelsüblicher 30% *wässriger Latexdispersion* von Poly(EA-MAA-TAMCL) in einer Auftragsmenge von 10% Trockensubstanz bezogen auf das Pelletbett (entspricht etwa 7 mg/cm$^2$) mit 20% Weichmacher bezogen auf das Polymer (z.B. TEC), und 50% Antiklebemittel bezogen auf das Polymer (z.B. Talk), erreicht.

So wird beispielsweise mit *lösungsmittelfreiem* Poly(EA-MAA-TAMCL)-*Pulver* (Eudragit® RS PO, üblicherweise als Tablettierhilfsstoff eingesetzt) bei erfindungsgemässer lösungsmittelfreier Verwendung ein Freisetzungsprofil gleicher Qualität erhalten. Um die dabei erhaltenen Freisetzungskurven zu vergleichen bzw. die Ähnlichkeit mit der Freisetzungen aus mit Eudragit RS 30 D befilmten Pellets bewerten zu können, wurde der Ähnlichkeitsfaktor bestimmt. Die Resultate, bzw. die erhaltenen Kurven, werden als vergleichbar angesehen, wenn der Ähnlichkeitsfaktor grösser als 50 ist. Dieser wird gemäss Gleichung 1 berechnet: Gleichung 1 (Berechnungsformel des Ähnlichkeitsfaktors):

$$f_2 = 50 * \log \left\{ \left[ 1 + \frac{\left( \sum_{t=1}^{n} R_t - T_t \right)^2}{n} \right]^{-0.5} * 100 \right\}$$

$f_2$ Ähnlichkeitsfaktor

$n$ Anzahl verglichener Messpunkte

$R_t$ Freigesetzte Menge der ersten Probe zum Zeitpunkt t

$T_t$ Freigesetzte Menge der zweiten Probe zum Zeitpunkt t

$t$ Zeitpunkt t

In Tabelle 1 sind die Ähnlichkeitsfaktoren der jeweiligen Freisetzungskurve aus mit verschiedenen Auftragsmengen handelsübliches Poly(EA-MAA-TAMCL)-Pulver (üblicherweise als Tablettierhilfsstoff eingesetzt) befilmten Pellets im Vergleich mit den mit den üblichen 10% (Trockensubstanz bezogen auf das Pelletbett = 7mg/cm$^2$) an handelsüblicher, 30%iger wässriger Latexdispersion von Poly(EA-MAA-TAMCL) (wässeriges Referenzverfahren) aufgelistet.

Tabelle 1

| Trockenbeschichtung mit Poly(EA-MMA-TAMCl)-Pulver (Auftrag in % bezogen auf das Pelletbett/ Auftrag in mg/cm$^2$) | Ähnlichkeitsfaktor $f_2$ im Vergleich mit 30%iger Poly(EA-MMATAMCl) - wässriger Latexdispersion (10% Auftrag Trockensubstanz = 7mg/cm$^2$, wässeriges Referenzverfahren) |
|---|---|
| 10%/7 | 33 |
| 15%/10 | 41 |
| 20%/14 | 70 |
| 25%/17 | 80 |

Dabei ist erkennbar, dass ein mit dem Referenz-Verfahren vergleichbares Freisetzungsprofil ab einem Auftrag von 20% handelsübliches Poly(EA-MAA-TAMCL)-Pulver erhalten wird. Es wurde auch festgestellt, dass das erfindungsgemässe Trockenbeschichtungsverfahren (solvent-free coating) mit mehrfach höheren Zuführungsraten durchgeführt werden kann als das entsprechende wässerige Referenzverfahren und somit wesentlich schneller ist als das Referenzverfahren. Das erfindungsgemässe Verfahren (im Vergleich zum Referenzverfahren) ist auch unter Berücksichtigung der von der Chargengrösse unabhängigen Verfilmungszeit von etwa 35 Minuten und der etwa doppelten Menge an aufzutragendem Polymer deutlich schneller.

Robustheit des Trockenbeschichtungsverfahrens: Die Robustheit eines Verfahrens wird danach bewertet, wie unbeeinflusst die Qualität des Produktes von Schwankungen kritischer Prozessparameter bleibt. Neben den maschinenkritischen Parametern (Filter, Beladung, Zuluftmenge bzw. Gutbewegung), die in der Vorevaluationen überprüft werden, verbleiben die Temperatur und Zeit, d.i. Beschichtungszeit und Filmbildungszeit (curing-time) als kritische Verfahrensparameter.

Für diese Untersuchungen wurden die Freisetzungsprofile von (a) mit 20% handelsübliches Poly(EA-MAA-TAMCL)-*Pulver* (entsprechend etwa 14mg/cm$^2$, üblicherweise als Tablettierhilfsstoff eingesetzt) gemäss dem erfindungsgemässen Verfahren befilmten Diclofenac-Pellets mit (b) 10% (Trockensubstanz bezogen auf das Pelletbett, entsprechend 7mg/cm$^2$) handelsüblicher 30%iger *wässriger* Latexdispersion von Poly(EA-MAA-TAMCL) befilmten Diclofenac-Pellets bei verschiedenen Temperaturen und nach verschiedenen Einwirkzeiten untersucht und über den Ähnlichkeitsfaktor verglichen. Je nach Zeit- bzw. Temperatureffekten, sind die Produktqualität und die Freisetzungsprofile oft deutlich verschieden. Es zeigte sich jedoch, dass auch bei starken Abweichungen der genannten Zeit- und Temperaturparameter gemäss dem erfindungsgemässen Verfahren die Ähnlichkeitsfaktoren ($f_2$) immer grösser 50 ($f_2>50$) waren, so dass die Freisetzungskurven jeweils nur sehr wenig voneinander verschieden waren. Das erfindungsgemässe Verfahren darf deshalb als robust bezeichnet werden. Dies im Experimentellen Teil hierin auch aufgezeigt.

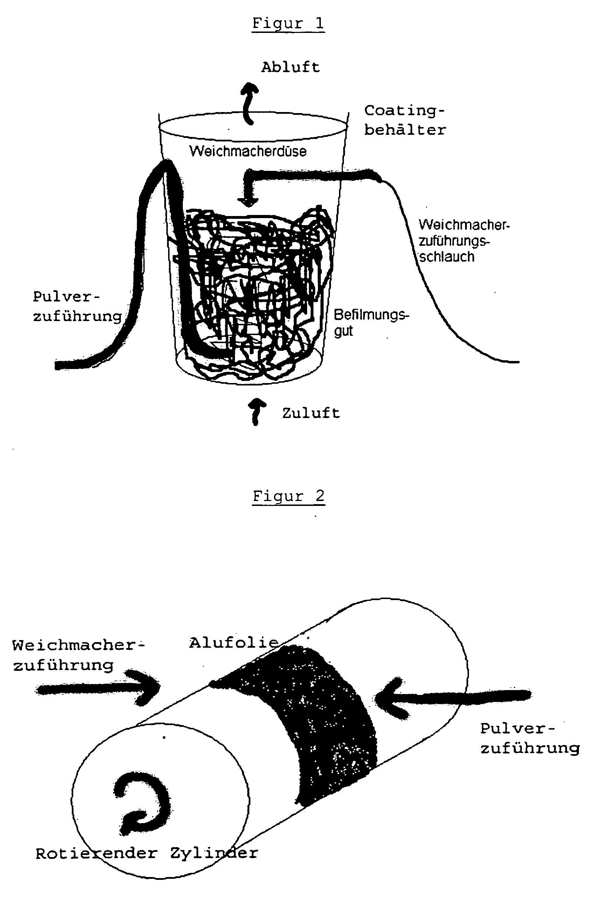

Die Beschichtungsapparatur: In Figur 1 ist die Beschichtungsapparatur schematisch dargestellt. Diese besteht aus dem Coating-Behälter (1) in welchen das Befilmungsgut (2) gegeben wird. Der Coating-Behälter (1) stellt schematisch die Apparatur zur Bildung der Wirbelschicht dar. Diese enthält die Pulverzuführung (3), die Leitung für die Zuluft (4), den Weichmacherzuführungsschlauch (5), welcher mit der Weichmacherdüse (6) versehen ist, sowie die Öffnung für die Abluft (7).

Die vorgängige Festlegung der optimalen Mengenverhältnisse des Polymers und des Weichmachers für die Beschichtung sowie der allfälligen zusätzlichen Komponenten, sowie der aufzubringenden Schichtdicke und der Temperatur und Zeitdauer, welche für die kohärente Filmbildung nötig sind, kann beispielsweise mit der in Figur 2 dargestellten Probenerstellungs-Apparatur erfolgen. Herzstück dieser Apparatur ist ein rotierender Zylinder auf den eine spezielle Alufolie aufgebracht ist. Auf dieser wird die Probe erstellt. Der Zylinder und damit die Folie "rotieren" an einer Weichmacher-Zuführungseinheit und einer Pulverzuführung vorbei, wodurch auf der Folie eine homogene Polymer-Weichmachermischung aufgezogen wird. Die Zuführung des flüssigen Weichmachers erfolgt über eine handelsübliche Sprühdüse. Die Pulverzuführung kann über unterschiedliche, an sich bekannte Zuführeinheiten/Apparaturen erfolgen, wie z.B. einen Schwingrinnendosierer, eine Corona-Pulverdüse, oder einen Schneckendosierer.

Über die Zeit und damit die Anzahl der Rotationsumläufe kann nun die "Schichtdicke" bzw. der flächenbezogene Auftrag variiert werden, während das Verhältnis Polymer zu Weichmacher über ein entsprechendes Verhältnis der jeweiligen Komponentenzuführungsraten eingestellt wird.

In einem ersten Schritt werden die Einstellungen der einzelnen Zuführungen, bei denen die gewünschten Auftragsraten erhalten werden können, evaluiert. Die verschiedenen Auftragsraten werden ermittelt, indem zunächst nur der Weichmacher auf die Filmziehfolie eine definierte Zeit aufgesprüht und durch Differenzwägung die entsprechende Rate berechnet wird. Anschliessend wird mit der gleichen Sprühdüseneinstellung zusätzlich und zeitlich parallel das Pulver eine definierte Zeit aufgebracht und durch Differenzwägung und Abzug der vorher ermittelten Weichmacherrate die entsprechende Pulverauftragsrate ermittelt.

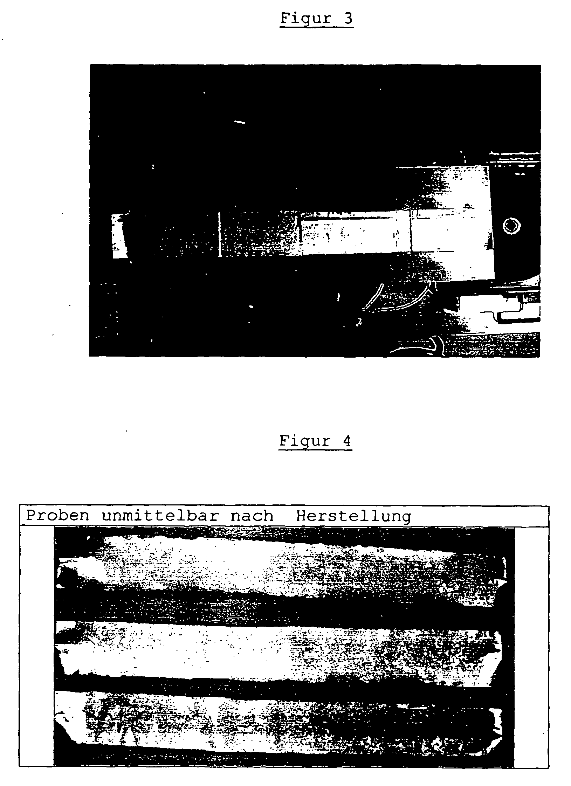

Die Apparatur für die Bestimmung der Minimalen Verfilmungstemperatur ist in Figur 3 aufgezeichnet. Auf einer massiven Alu-Platte wird ein konstanter TemperaturGradienten erzeugt. Dies geschieht dadurch, dass eine Alu-Platte auf der einen Seite mit konstanter Heizrate erwärmt wird, am anderen Ende durch Wasserkühlung gekühlt wird. Durch diesen Temperaturunterschied zwischen den beiden Plattenenden wird ein Wärmestrom vom warmen zum kalten Seite induziert, was nach einer Einpendelphase zu einem konstanten Temperaturgradienten führt. Das Verhalten der oben auf der Alufolie erstellten Polymerpulver-/Weichmacherproben (siehe Kommentar zu Figur 2) wird nach Fixierung der Probe auf der Alu-Platte beobachtet, die somit die Minimale Temperatur, d.i. die Minimale Verfilmungstemperatur (MVT), anzeigt, bei der eine Verfilmung statt findet.

Bei den lösungsmittelfreien Proben wurden die auf Folien, nach obigen Verfahren (Figur 2) aufgezogenen Polymer/- Weichmacher-Mischungen auf der Heizplatte gemäss Figur 3 aufgebracht. Um einen optimalen Kontakt der Folie mit der Heizplattenoberfläche zu gewährleisten, werden die Folien vorzugsweise mit Silikonöl auf der Heizplatte fixiert.

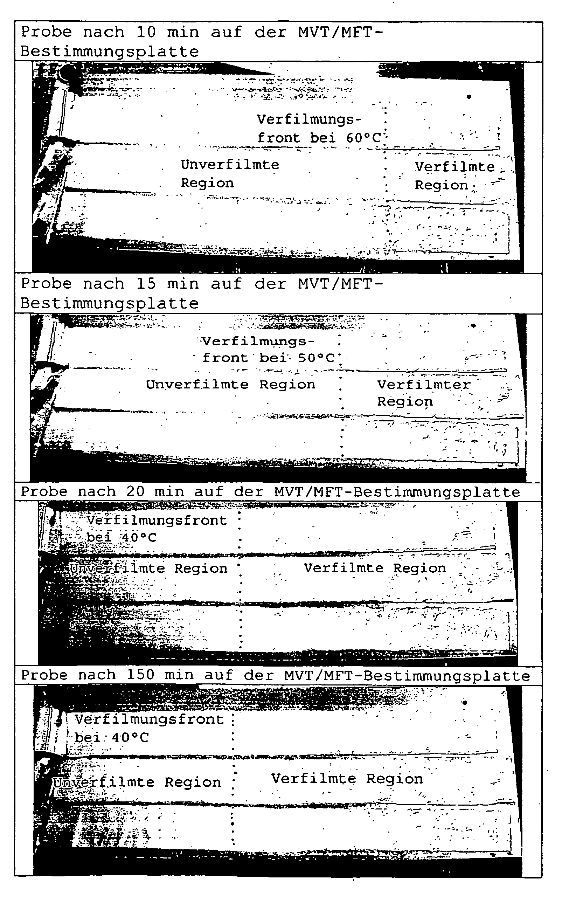

Als Beispiel wird hier das Verhalten einer Probe aus dem Filmbildnerpolymerpulver Poly(EA-MMA-TAMCl) (14 mg/cm2) + 40% TEC in Figur 4 gezeigt. Die folgende Beispiele erläutern die Erfindung.

Beispiel 1

Unbeschichtete Diclofenac-Pellets (Kerne) werden in einer Wirbelschicht gemäss Figur 1 mit einer Polymer/ Talk-Mischung von 1:1 + 40 % TEC gemäss den in Tabelle 2 angegebenen Mengen und Verfahrensparametern beschichtet.

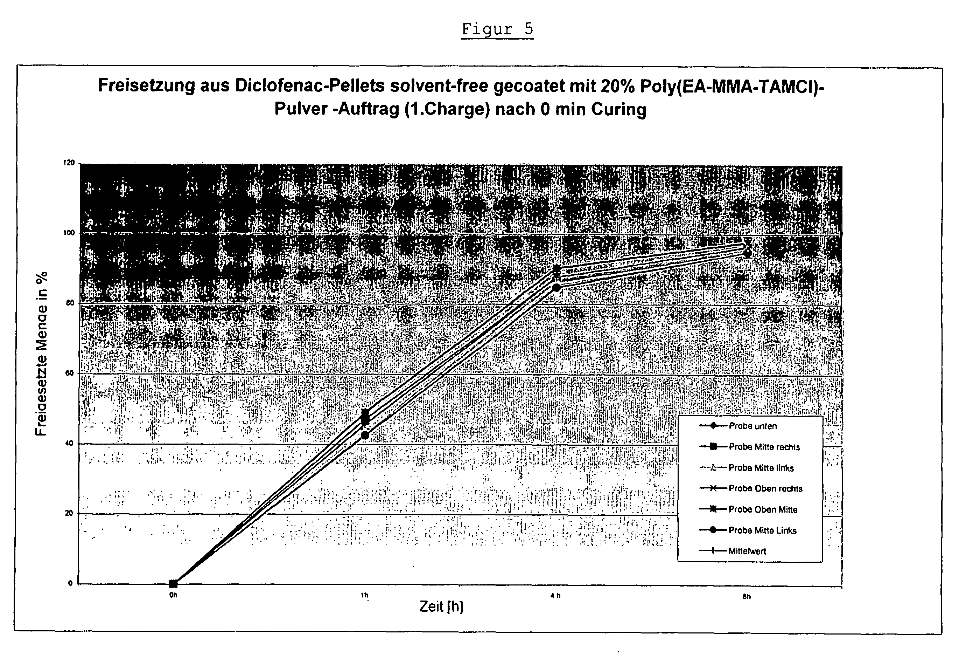

Gemäss Beispiel 1 werden ausgezeichnete Filme mit sehr gutem Freisetzungsprofil erhalten, wie dies in Figur 5 gezeigt ist.

Tabelle 2

| Material | Menge |
|---|---|
| Diclofenac-Pellets | 2000 g |

(fortgesetzt)

| Material | Menge |
|---|---|
| Polymer/Talk-Mischung 1:1 Poly(EA-MMA-TAMCl-Pulver | Je nach Auftragsmenge (10-25% bezogen auf Pelletbett) 400-1000 g |
| Triethylcitrat - Weichmacher | 40% bezogen auf das Polymer |
| Coating - Verfahrensparameter | |
| Zulufttemperatur | 50-60°C |
| Kernbett-Temperatur | 45°-50C |
| Sprühdüsendurchmesser | 1,0 mm |
| Zuluftmenge | 30-70 m3/h |
| Pulverzuführungsdruck | 3 bar |
| Sprühdruck | 1 bar |
| Sprührate | 2.6 g/min |
| Pulverzuführungsrate | 13 g/min |
| Prozessdauer | Je nach Auftragsmenge |
| Verfilmung | |
| Zulufttemperatur | 60°C |
| Abluft | 45°C |
| Zuluftmenge | 50 m3/h |
| Zeit | 35 min |

Tabelle 3 (siehe Beispiele 2-21)

| Bsp. Nr. | Polymer jeweils 100 Teile | Weichmacher/ Teile | Hilfsstoffe Teile | Total-Auftrag Polymer mg/cm$^2$ |
|---|---|---|---|---|
| 2 | MC | Myvacet/40 | Talk/ 100 Citronearoma/ 2 | 17 |
| 3 | HPMC | ATBC/35 | Talk/ 100 PVA (Kleber)/ 2 | 16 |
| 4 | CAP | ATEC /45 | Citronearoma/ 1 Chinolingelb/ 2 | 19 |
| 5 | HPMCMP | ATEC/ 35 | Talk/ 50 Lactoflavin/ 2 | 22 |
| 6 | EC | DEP/ 30 | Curcumin/ 2 | 21 |
| 7 | HPC | TBC/ 25 | Talk/ 100 | 19 |
| 8 | HPMC | DMP/ 20 | Talk/ 50 PVA/ 2 | 15 |
| 9 | HEC | DBS/ 30 | Amaranth/ 2 | 27 |
| 10 | HPMCP | Glycerin/ 20 | Talk/ 120 PVA (Kleber)/ 2 | 20 |
| 11 | Poly-MA-EA | DBS/ 20 | - | 16 |
| 12 | Poly-MA-EA | DEP/ 35 | Talk/ 60 | 19 |
| 13 | Poly-MA-EA | TEC/ 40 | Talk/ 100 Curcumin/ 1 | 18 |
| 14 | [Poly(MA-MMA)] | DBS/ 25 | Talk/ 100 | 14 |
| 15 | [Poly(MA-MMA)] | DEP/ 35 | Talk/ 100 | 17 |

(fortgesetzt)

| Bsp. Nr. | Polymer jeweils 100 Teile | Weichmacher/ Teile | Hilfsstoffe Teile | Total-Auftrag Polymer mg/cm$^2$ |
|---|---|---|---|---|
| 16 | [Poly(EA-MMA-TAMCl)] | TEC/ 40 | Talk/ 100 Citronearoma/ 1 Chinolingelb/ 2 | 45 |
| 17 | [Poly(EA-MMA-TAMCl)] | DEP/ 30 | Talk/ 90 | 4 |
| 18 | [Poly(EA-MMA-TAMCl)] | DBS/ 30 | Talk/ 80 | 48 |
| 19 | PVAc | Glycerin-triacetat/ 45 | Talk/ 45 | 17 |
| 20 | PVP | DES /35 | Talk/ 100 Citronearoma/ 1 Chinolingelb/ 2 | 22 |
| 21 | PVAP | ATEC/ 20 | Talk/ 100 Citronearoma/ 1 Amaranth/ 2 | 9 |

Beispiele 2-21

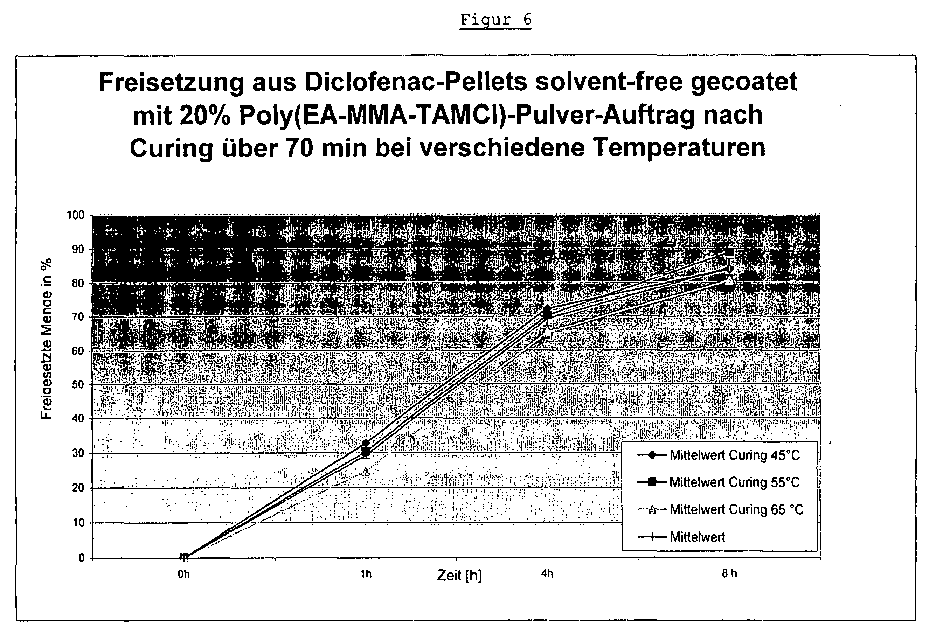

Analog zu Beispiel 1 wurden die in Tabelle 3 angegebenen Zusammensetzungen in der erfindungsgemässen Trockenbeschichtung auf unbeschichtete Diclofenac-Pellets (Kerne) in einer Wirbelschicht aufgebracht.
In den Beispielen 2-21 wurden ähnliche optimale Eigenschaften erhalten, wie in den Figur 5 (siehe Beispiel 1) und Figur 6 (siehe Beispiel 22) gezeigt.

Beispiel 22 (Freisetzungsprofil)

Von der gemäss Beispiel 1 beschichtete Charge wurde die Homogenität der Befilmung bewertet bzw. das Freisetzungsprofil gemessen. Hierzu wurden je eine Probe von Mitte unten, Mitte rechts, Mitte links, Oben rechts, Oben links und oben Mitte gezogen und deren Freisetzung für die Untersuchung herangezogen. Die Resultate sind in Tabelle 4 aufgelistet.

Tabelle 4 (Freisetzung nach Beschichtung bei 45°C; Curing-Zeit 35 min)

| Soll-Einwaage (berechnet aus Kapselfüllgewicht unbefilmte Pellets (300 mg) + Filmauftrag) | | 444 mg (421.18-466.2 = +/-5% (wie spezifiziert) | | |
|---|---|---|---|---|
| Probennummer | Einwaage Pellets in mg | Freigesetzte Menge in % über die Zeit | | |
| | | 1 h | 4 h | 8 h |
| Spezifikation | | 15-45 % | 55-85 % | Grösser gleich 80% |
| Probe 1 | 446.6 | 49.078 | 90.224 | 97.652 |
| Probe 2 | 437.4 | 47.143 | 87.588 | 96.241 |
| Probe 3 | 447.2 | 43.852 | 85.765 | 99.758 |
| Probe 4 | 447.4 | 42.818 | 86.940 | 96.720 |
| Probe 5 | 443.4 | 48.983 | 90.157 | 97.578 |
| Probe 6 | 447.1 | 42.485 | 84.697 | 94.764 |
| Mittelwert | - | 45.727 | 87.562 | 96.286 |
| Relative Standardabweichung | - | 6.7 | 2.6 | 1.3 |

Die Homogenität der Befilmung, die Robustheit und Reproduzierbarkeit des Verfahren gemäss Beispiel 22 ergab analoge ausgezeichnete Resultate, wie solche in Beispiel 1 (siehe Figur 5) erhalten wurden.
Auch die Robustheit des Verfahrens gegenüber dem kritischen Parameter Temperatur für die Herstellung von im Trockenbeschichtungsverfahren hergestellten Pellets, beschichtet mit 20%-Poly(EA-MMA-TAMCl)-Pulver (bezogen auf das Pelletbett = 14 mg/cm$^2$) Auftrag, mit 40% TEC, wurde untersucht. Exemplarisch sei der kritische Prozessparameter "Temperatur" aufgezeigt. Die unter Beispiel 1 be-filmten Pellets wurden Temperaturbelastungen von 45°C, 55°C und 65 °C über 70 Minuten unterworfen und anschliessend die Freisetzungsprofile anhand des Ähnlichkeitsfaktors untersucht (Figur 6 und folgende Tabelle 5).

Tabelle 5

|  | Mittelwert Freisetzung 45°C [%] | Mittelwert Freisetzung 55°C [%] | Mittelwert Freisetzung FS 65°C [%] | Mittelwert [%] | Standard-abweichung | Rel. Standard-abweichung |
|---|---|---|---|---|---|---|
| 0h | 0 | 0 | 0 | 0 | 0 | #DIV/0! |
| 1h | 32.8333333 | 30.3333333 | 24.6666667 | 29.2777778 | 4.18440668 | 14.3% |
| 4h | 72.3333333 | 70.3333333 | 65.3333333 | 69.3333333 | 3.60555128 | 5.2% |
| 8h | 84 | 89 | 80.6666667 | 84.5555556 | 4.19435246 | 5.0% |

Die Ähnlichkeitsfaktoren lagen immer weit über 50 (Vgl. 45°C und 55°C: $F_2$ 79; vgl. 45°C und 65°C; $F_2$ =59; vgl. 55 und 65°C: $F_2$ 59), so dass die Freisetzungsprofile als signifikant ähnlich bewertet werden können, damit ein Einfluss des Parameters "Temperatur" als gering eingeschätzt werden kann und daher das Verfahren als gegenüber diesem Parameter robust angesehen werden darf.

Auch die Reproduzierbarkeit der Herstellung von im Trockenbeschichtungsverfahren hergestellten Pellets, beschichtet mit Poly(EA-MMA-TAMCI)-Pulver, 20%-Auftrag, mit 40% TEC, wurde untersucht. Die Zuführung der Pulver-bzw. Weichmacherkomponente erfolgte bei allen Versuchen gut reproduzierbar und ohne Verstopfung der Düse bzw. des Pulverzuführungschlauches. Bei keinem der Versuche konnte eine Agglomeratbildung festgestellt werden. Die Coatingeffektivität war bei allen Versuchen etwa gleich hoch (etwa 90%).

Die Reproduzierbarkeit der Herstellung bezüglich der Freisetzung wurde anhand des Streuung bzw. der relativen Standardabweichung der chargenbezogenen Mittelwerte um die über diese gemittelten Werte ermittelt. Die Reproduzierbarkeit wurde dann als gewährleistet angesehen, wenn die für das wässrigen Verfahren mit handelsüblicher 30%iger wässriger Latex aus Poly(EA-MMA-TAMCI) ermittelten Grenzen von S rel. = +/-15 % eingehalten wurde. Die erhaltenen Resultate sind in Tabelle 6 wieder gegeben und zeigen eine hohe Reproduzierbarkeit.

Tabelle 6 (nach Curing bei 45°C während 35 Minuten)

|  | Mittelwert Charge 1 | Mittelwert Charge 2 | Mittelwert Charge 3 | Mittelwert | S | S rel. |
|---|---|---|---|---|---|---|
| 0h | 0 | 0 | 0 | 0 | 0 | #DIV/0! |
| 1 h | 45.7265 | 42.7333333 | 38.8333333 | 42.4310556 | 3.45651061 | 8.1% |
| 4 h | 87.5618333 | 82.9246667 | 73.8333333 | 81.4399444 | 6.98364003 | 8.6% |
| 8 h | 96.2855 | 93.3846667 | 86.8333333 | 92.1678333 | 4.84214583 | 5.3% |

**Patentansprüche**

1. Trockenbeschichtungsverfahren für feste Substrate, insbesondere für die Herstellung von beschichteten pharmazeutischen Darreichungsformen, **dadurch gekennzeichnet, dass**

(i) ausschliesslich Beschichtungskomponenten zur Anwendung kommen, welche aus (a) mindestens einem Beschichtungspolymer und (b) mindestens einem bei der Beschichtungstemperatur flüssigen Weichmacher bestehen;
(ii) die beiden Komponenten (a) und (b) separat auf die Kerne in dem vorgängig bestimmten Mengenverhältnis und der vorgängig bestimmten Schichtdicke aufgebracht werden und bei der für die kohärente Filmbildung vorgängig bestimmten Temperatur und Zeitdauer so lange gehalten werden, bis sich ein kohärenter Film gebildet hat; wobei
(iii)das Beschichtungspolymer ausgewählt ist aus der Gruppe enthaltend Cellulosederivate, Polyacrylate und Polyvinylderivate, und
(iv) der Weichmacher ausgewählt ist aus der Gruppe enthaltend acetylierte Fettsäureglyceride, Citrate, Phtalate, Sebacate, Glycerin und Glycerinderivate, Polyoxyethylen-Polyoxypropylen-Copolymere, Propylengykole und Rizinusöl, und

(v) man höchstens 20 Gew.-% Lösungsmittel, bezogen auf das Kerngewicht, mit dem Weichmacher oder separat, zur Beschichtung aufträgt.

2. Verfahren nach Anspruch 1, für die Herstellung von enterisch und/oder retardierend und/oder schützend und/- oder kosmetisch wirkenden beschichteten Darreichungsformen, vorzugsweise von beschichteten Tabletten, Mikrotabletten, feste Lösungen, Kapseln und Pellets.

3. Verfahren nach Anspruch 1 oder 2, **dadurch gekennzeichnet, dass** die Beschichtungskomponenten weitere Hilfsstoffe enthalten, ausgewählt aus der Gruppe enthaltend Bindemittel, Trennmittel/Antiklebemittel, Geschmacksstoffe und Farbstoffe.

4. Verfahren nach einem der Ansprüche 1-3, **dadurch gekennzeichnet, dass** verwendete Bindemittel vorzugsweise HPMC, PVP oder PVA; Trennmittel/Antiklebemittel vorzugsweise Talk oder Syloid; synthetische oder natürliche Geschmacksstoffe vorzugsweise Orangen-, Minz-, Zitronenaromen; und natürliche oder synthetische Farbstoffe vorzugsweise Curcurmin, Lactoflavin, Chinolingelb oder Amaranth, darstellen.

5. Verfahren nach einem der Ansprüche 1-4, **dadurch gekennzeichnet, dass** die weiteren festen Hilfsstoffe neben dem Beschichtungspolymer separat zugesetzt werden oder vorgängig in dieses eingearbeitet sind und die weiteren flüssigen Hilfsstoffe mit dem Weichmacher gemeinsam auf die Oberfläche des Substratsaufgebracht werden.

6. Verfahren nach einem der Ansprüche 1-5, **dadurch gekennzeichnet, dass** höchstens 15 Gew.-% Lösungsmittel, vorzugsweise Wasser, vorzugsweise höchstens 10 Gew.-% Lösungsmittel, vorzugsweise höchstens 5 Gew.-% Lösungsmittel, vorzugsweise höchstens 2 Gew.-% Lösungsmittel, und vorzugsweise höchstens 1-1.5 Gew.-% Lösungsmittel, jeweils bezogen auf das Kerngewicht, im Beschichtungsverfahren, mit dem Weichmacher oder separat, vorzugsweise am Ende des Beschichtungs vorganges, zur Beschichtung aufgetragen wesden.

7. Verfahren nach einem der Ansprüche 1-5, **dadurch gekennzeichnet, dass** den Beschichtungskomponenten kein Lösungsmittel zugesetzt oder nach der Beschichtung aufgetragen wird.

8. Verfahren nach einem der Ansprüche 1-7, **dadurch gekennzeichnet, dass** die Cellulosederivate ausgewählt sind aus der Gruppe enthaltend Celluloseether und Celluloseester, vorzugsweise Methylcellulose (MC), Hydroxypropylmethylcellulose (HPMC), Celluloseacetatphathalat (CAP), Hydroxypropylmethylcellulosemethylphthalat (HPMCMP), Ethylcellulose (EC), Hydroxypropylcellulose (HPC), Hydroxypropylmethylcellulose (HPMC), Hydroxyethylmethylcellulose (HEC), Carboxymethylethylcellulose und Salze (CMEC), Carboxymethylcellulose und ihre Salze (CMC), Hydroxyethylcellulose (HEC), Hydroxypropylmethylcellulosephthalat (HPMCP), Hydroxypropylmethylcelluloseacetatsuccinat (HPMCAS).

9. Verfahren nach einem der Ansprüche 1-7, **dadurch gekennzeichnet, dass** die Polyacrylate ausgewählt sind aus der Gruppe enthaltend Methacrylsäure-Copolymere, Aminoalkylmethacrylate, und Metharylester-Copolymere, vorzugsweise Poly(ethylacrylat, methylacrylsäure) (Poly-MA-EA), Poly(methylmethacrylat, methacrylsäure) [Poly-(MA-MMA)], Poly[butylmethacrylat, (2-dimethylaminoethyl)methacrylat, methylmethacrylat] [Poly(BMA-DAEM-MMA)], Poly(ethylacrylat, methylmethacrylat, trimethylammonioethylmethacrylatchlorid) [Poly(EA-MMA-TAMCl)] und Poly (ethylacrylat, methylmethacrylat) [Poly(EA-MMA)].

10. Verfahren nach einem der Ansprüche 1-7, **dadurch gekennzeichnet, dass** die Polyvinylderivate ausgewählt sind aus der Gruppe enthaltend Polyvinylacetat-Copolymere und Polyvinylpyrrolidone, vorzugsweise Vinylacetat-Vinylpyrrolidon-Copolymer (PVAc), Polyvinylactatphathalat (PVAP) und Polyvinylpyrrolidon (PVP).

11. Verfahren nach einem der Ansprüche 1-10, **dadurch gekennzeichnet, dass** die Weichmacher ausgewählt sind aus der Gruppe enthaltend acetylierten Fettsäuregliceriden, vorzugsweise Myvacet K-9-45; Citrate, vorzugsweise Acetyltributylcitrat (ATBC), Acetyltriethylcitrat (ATEC), Tributylcitrat (TBC) und Triethylcitrat (TEC); Phthalate, vorzugsweise Dibutylphathalat (DBP), Diethylphthalat (DEP) und Dimethylphtalat (DMP); Sebacate, vorzugsweise Diethylsebacat (DES) und Dibutylsebacat (DBS); Glycerin; Glycerintriacetat; und Polyethylenglykolpropoxylat.

12. Verfahren nach einem der Ansprüche 1-11, **dadurch gekennzeichnet, dass** man 1-50 mg/cm$^2$ an Beschichtungspolymer, vorzugsweise 4-25 mg/cm$^2$ aufträgt und das Gewichtsverhältnis von Weichmacher:Beschichtungspolymer 1:50 bis 1:1, vorzugsweise 1:40 bis 2:5, beträgt.

**13.** Verfahren nach einem der Ansprüche 1-12, **dadurch gekennzeichnet, dass** das Gewichtsverhältnis der Summe aller weiterer Hilfsstoffe:Beschichtungspolymer 1:50 bis 1:1, vorzugsweise 1:40 bis 2:5, beträgt.

**14.** Verfahren nach einem der Ansprüche 1-12, **dadurch gekennzeichnet, dass** die Temperatur der Beschichtungskomponenten beim Aufbringen auf die Kerne im Bereich von 45-70°C, vorzugsweise im Bereich von 50-65°C, vorzugsweise bei 50-60°C, liegt.

**15.** Verfahren nach einem der Ansprüche 1-14, **dadurch gekennzeichnet, dass** die Temperatur der zu beschichtenden Kerne in der Wirbelschicht im Bereich von 20-55°C, vorzugsweise im Bereich von 40-55°C und insbesondere im Bereich von 45-50°C, liegt.

**16.** Verfahren nach einem der Ansprüche 1-15, **dadurch gekennzeichnet, dass** die Beschichtung nach dem Auftrag der gesamten Auftragsmenge bei der Auftragstemperatur während 30 Minuten bis 120 Minuten, vorzugsweise 60 Minuten bis 90 Minuten belassen wird.

**17.** Verfahren nach einem der Ansprüche 1-16, **dadurch gekennzeichnet, dass** die für Schritt (ii) nach Anspruch 1 nötigen optimierten Mengenverhältnisse der Komponenten, die bildende Schichtdicke, sowie die optimale Temperatur und Zeitdauer für die kohärente Filmbildung, bzw. die zur Probenerstellung und MVT-Bestimmungsverfahren, zur Ermittlung der Minimalen Verfilmungstemperatur bzw. Minimalen Verfilmungszeit, vorgängig zum Materialauftrag bestimmt werden.

**Claims**

**1.** A dry-coating method for solid substrates, in particular for producing coated pharmaceutical administration forms, **characterized in that**

(i) use is made exclusively of solvent-free coating components which consist of (a) at least one coating polymer and (b) at least one plasticizer which is liquid at the coating temperature;
(ii) the two components (a) and (b) are applied separately to the core in the previously determined quantity ratio and the previously determined coating thickness and are maintained at the previously determined temperature and duration for the coherent formation of a film until a coherent film has formed; wherein
(iii) the coating polymer is selected from the group containing cellulose derivatives, polyacrylate and polyvinyl derivatives, and
(iv) the plasticizer is selected from the group containing acetylated fatty acid glycerides, citrates, phthalates, sebacates, glycerine and glycerine derivatives, polyoxyethylene-polyoxypropylene copolymers, propylene glycols and castor oil, and
(v) a maximum of 20 weight-% of solvent referred to the core weight is applied, with the plasticizer or separately, as a coating.

**2.** The method as claimed in claim 1 for the production of coated administration forms with enteric and/or retarding and/or protective and/or cosmetic action, preferably of coated tablets, micro-tablets, solid solutions, capsules and pellets.

**3.** The method as claimed in claim 1 or 2, **characterized in that** the coating components contain further excipients selected from the group containing bonding agents, separating agents/anti-caking agents, flavor additives and colorants.

**4.** The method as claimed in one of claims 1-3, **characterized in that** bonding agents used preferably constitute HPMC, PVP or PVA; separating agents/anti-caking agents preferably constitute talc or syloid; synthetic or natural flavor additives preferably constitute orange, mint or lemon flavors; and natural or synthetic colorants preferably constitute curcurmin, lactoflavin, chinolin yellow or amaranth.

**5.** The method as claimed in one of claims 1-4, **characterized in that** the further solid excipients are added separately along with the coating polymer or are previously incorporated therein, and the further liquid excipients are applied together with the plasticizer to the surface of the substrate.

**6.** The method as claimed in one of claims 1-5, **characterized in that** a maximum of 15 weight-% of solvent, preferably water, preferably a maximum of 10 weight-% of solvent, preferably a maximum of 5 weight-% of solvent, preferably a maximum of 2 weight-% of solvent, and preferably a maximum of 1-1.5 weight-% of solvent, in each case referred to the core weight, are applied in the coating method, with the plasticizer or separately, to form a coating, preferably at the end of the coating process.

7. The method as claimed in one of claims 1-5, **characterized in that** no solvent is added to the coating components or applied after the coating.

8. The method as claimed in one of claims 1-7, **characterized in that** the cellulose derivatives are selected from the group containing cellulose ether and cellulose ester, preferably methyl cellulose (MC), hydroxypropyl methylcellulose (HPMC), cellulose acetate phthalate (CAP), hydroxypropyl methylcellulose methylphthalate (HPMCMP), ethyl cellulose (EC), hydroxypropyl cellulose (HPC), hydroxypropyl methylcellulose (HPMC), hydroxyethyl methyl cellulose (HEC), carboxymethyl ethyl cellulose and salts (CMEC), carboxymethyl cellulose and its salts (CMC), hydroxyethyl cellulose (HEC), hydroxypropyl methyl cellulose phthalate (HPMCP), hydroxypropyl methyl cellulose acetate succinate (HPMCAS).

9. The method as claimed in one of claims 1-7, **characterized in that** the polyacrylates are selected from the group containing methacrylic acid copolymers, aminoalkyl methacrylates, and methacrylic ester copolymers, preferably poly(ethyl acrylate, methylacrylic acid) (poly-MA-EA), poly(methyl methacrylate, methacrylic acid) [poly-(MA-MMA)], poly[butylmethacrylate, (2-dimethylaminoethyl)methacrylate, methyl methacrylate] [poly(BMA-DAEM-MMA)], poly (ethyl acrylate, methyl methacrylate, trimethylammonium ethyl methacrylate chloride) [poly(EA-MMA-TAMC1)] and poly(ethyl acrylate, methyl methacrylate) [poly(EA-MMA)].

10. The method as claimed in one of claims 1-7, **characterized in that** the polyvinyl derivatives are selected from the group containing polyvinyl acetate copolymers and polyvinylpyrrolidones, preferably vinyl acetate vinyl pyrrolidone copolymer (PVAc), polyvinyl acetate phthalate (PVAP) and polyvinylpyrrolidone (PVP).

11. The method as claimed in one of claims 1-10, **characterized in that** the plasticizer is selected from the group containing acetylated fatty acid glycerides, preferably Myvacet K-9-45; citrates, preferably acetyl tributyl citrate (ATBC), acetyl triethyl citrate (ATEC), tributyl citrate (TBC) and triethyl citrate (TEC); phthalates, preferably dibutyl phthalate (DBP), diethyl phthalate (DEP) and dimethyl phthalate (DMP); sebacates, preferably diethyl sebacate (DES) and dibutyl sebacate (DBS); glycerine; glycerin triacetate; and polyethylene glycol propoxylate.

12. The method as claimed in one of claims 1-11, **characterized in that** $1-50$ $mg/cm^2$ of coating polymer, preferably $4-25$ $mg/cm^2$, are applied, and the weight ratio of plasticizer to coating polymer is 1:50 to 1:1, preferably 1:40 to 2:5.

13. The method as claimed in one of claims 1-12, **characterized in that** the weight ratio of the sum of all further excipients to coating polymer is 1:50 to 1:1, preferably 1:40 to 2:5.

14. The method as claimed in one of claims 1-12, **characterized in that** the temperature of the coating components when applied to the core lies in the range from 45-70°C, preferably in the range from 50-65°C, preferably at 50-60°C.

15. The method as claimed in one of claims 1-14, **characterized in that** the temperature of the cores to be coated in the fluidized bed lies in the range from 20-55°C, preferably in the range from 40-55°C, and in particular in the range from 45-50°C.

16. The method as claimed in one of claims 1-15, **characterized in that**, after the application of the total application quantity, the coating is left at the application temperature for 30 minutes to 120 minutes, preferably 60 minutes to 90 minutes.

17. The method as claimed in one of claims 1-16, **characterized in that** the optimized quantity ratios of the components required for step (ii) as claimed in claim 1, the forming coating thickness, and the optimum temperature and duration for the formation of a coherent film, or the process for producing the sample and determining the MFFT, are determined prior to applying the material in order to determine the minimum film formation temperature and minimum film formation time.

## Revendications

1. Procédé de revêtement à sec pour substrats solides, en particulier pour la fabrication de formes galéniques enrobées, **caractérisé en ce que** :

    (i) les composants d'enrobage utilisés sont exclusivement (a) au moins un polymère d'enrobage et (b) au moins un plastifiant liquide à la température d'enrobage ;
    (ii) les deux composants (a) et (b) sont appliqués séparément sur les noyaux, dans un rapport quantitatif préalablement déterminé et avec une épaisseur de couche préalablement déterminée, et sont maintenus à la température préalablement déterminée pour la formation d'un film cohérent pendant la durée nécessaire pour que se forme un film cohérent ; dans lequel :
    (iii) le polymère d'enrobage est choisi dans le groupe comprenant les dérivés de cellulose, les polyacrylates et les dérivés polyvinyliques ; et
    (iv) le plastifiant est choisi dans le groupe comprenant les glycérides d'acides gras acétylés, les citrates, les phtalates, les sébaçates, le glycérol et les dérivés de glycérol, les copolymères polyoxyéthylène-polyoxypropylène, les propylène glycols et l'huile de ricin ; et **en ce que**
    (v) l'on applique pour l'enrobage au plus 20 % en poids de solvant, par rapport au poids du noyau, avec le plastifiant ou séparément.

2. Procédé selon la revendication 1, pour la fabrication de formes galéniques enrobées à action entérique et/ou retardée et/ou de protection et/ou cosmétique, de préférence de comprimés, de microcomprimés, de solutions solides, de capsules et de cachets enrobés.

3. Procédé selon l'une des revendications 1 ou 2, **caractérisé en ce que** les composants d'enrobage contiennent d'autres adjuvants, choisis dans le groupe comprenant des liants, des agents de démoulage/de séparation, des arômes et des colorants.

4. Procédé selon l'une des revendications 1 à 3, **caractérisé en ce que** les liants utilisés sont, de préférence, HPMC, PVP ou PVA ; les agents de démoulage/de séparation sont, de préférence, le talc ou le syloïde ; les arômes synthétiques ou naturels sont, de préférence, les arômes d'orange, de menthe et de citron ; et les colorants naturels ou synthétiques sont, de préférence, la curcumine, la lactoflavine, le jaune de quinoléine ou l'amarante.

5. Procédé selon l'une des revendications 1 à 4, **caractérisé en ce que** les autres adjuvants solides outre le polymère d'enrobage sont ajoutés séparément ou incorporés au préalable dans celui-ci et **en ce que** les autres adjuvants liquides sont appliqués en même temps que le plastifiant à la surface du substrat.

6. Procédé selon l'une des revendications 1 à 5, **caractérisé en ce que** l'on applique pour l'enrobage au plus 15 % en poids de solvant, de préférence l'eau, de préférence au plus 10 % en poids de solvant, de préférence au plus 5 % en poids de solvant, de préférence au plus 2 % en poids de solvant et de préférence au plus 1 à 1,5 % en poids de solvant, toujours par rapport au poids du noyau, dans le procédé d'enrobage, avec le plastifiant ou séparément, de préférence à la fin de l'opération d'enrobage.

7. Procédé selon l'une des revendications 1 à 5, **caractérisé en ce qu'**aucun solvant n'est ajouté aux composants d'enrobage ou appliqué après l'enrobage.

8. Procédé selon l'une des revendications 1 à 7, **caractérisé en ce que** les dérivés de cellulose sont choisis dans le groupe comprenant les éthers de cellulose et les esters de cellulose, de préférence la méthylcellulose (MC), l'hydroxypropylméthylcellulose (HPMC), l'acétate phtalate de cellulose (CAP), le méthylphtalate d'hydroxypropylméthylcellulose (HPMCMP), l'éthylcellulose (EC), l'hydroxypropylcellulose (HPC), l'hydroxypropylméthylcellulose (HPMC), l'hydroxyéthylméthylcellulose (HEC), la carboxyméthyléthylcellulose et ses sels (CMEL), la carboxyméthylcellulose et ses sels (CMC), l'hydroxyéthylcellulose (HEC), le phtalate d'hydroxypropylméthylcellulose (HPMCP), l'acétate succinate d'hydroxypropylméthylcellulose (HPMCAS).

9. Procédé selon l'une des revendications 1 à 7, **caractérisé en ce que** les polyacrylates sont choisis dans le groupe comprenant les copolymères d'acide méthacrylique, les méthacrylates d'aminoalkyle et les copolymères d'ester méthacrylique, de préférence le poly(acrylate d'éthyle, acide méthylacrylique) (Poly-MA-EA), le poly(acrylate de méthyle, acide méthylacrylique) [Poly-(MA-MMA)], le poly[méthacrylate de butyle, le méthacrylate de (2-diméthylaminoéthyle), méthacrylate de méthyle] [Poly(BMA-DAEM-MMA)], le poly(acrylate d'éthyle, méthacrylate de méthyle, chlorure de triméthylammonioéthylméthacrylate) [Poly(EA-MMA-TAMCl)] et le poly(acrylate d'éthyle, méthacrylate de méthyle) [Poly(EA-MMA)].

10. Procédé selon l'une des revendications 1 à 7, **caractérisé en ce que** les dérivés polyvinyliques sont choisis dans le groupe comprenant les copolymères de poly(acétate de vinyle) et les polyvinylpyrrolidones, de préférence le copolymère acétate de vinyle-vinylpyrrolidone (PCAc), le poly(lactate phtalate de vinyle) (PVAP) et le polyvinylpyrrolidone (PVP).

11. Procédé selon l'une des revendications 1 à 10, **caractérisé en ce que** les plastifiants sont choisis dans le groupe comprenant les glycérides d'acides gras acétylés, de préférence le Myvacet K-9-45 ; les citrates, de préférence l'acétylcitrate de tributyle (ATBC), l'acétylcitrate de triéthyle (ATEC), le citrate de tributyle (TBC) et le citrate de triéthyle (TEC) ; les phtalates, de préférence le phtalate de dibutyle (DBP), le phtalate de diéthyle (DEP) et le phtalate de diméthyle (DMP) ; les sébaçates, de préférence le sébaçate de diéthyle (DES) et le sébaçate de dibutyle (DBS) ; le glycérol ; le triacétate de glycérol ; et le propoxylate de polyéthylène glycol.

12. Procédé selon l'une des revendications 1 à 11, **caractérisé en ce que** l'on applique 1 à 50 mg/cm$^2$ de polymère d'enrobage, de préférence 4 à 25 mg/cm$^2$, et **en ce que** le rapport en poids entre le plastifiant et le polymère d'enrobage est de 1:50 à 1:1, de préférence de 1:40 à 2:5.

13. Procédé selon l'une des revendications 1 à 12, **caractérisé en ce que** le rapport en poids entre la somme de tous les autres adjuvants et le polymère d'enrobage est de 1:50 à 1:1, de préférence de 1:40 à 2:5.

14. Procédé selon l'une des revendications 1 à 12, **caractérisé en ce que** la température des composants d'enrobage, lors de l'application sur les noyaux, se situe dans la plage de 45 à 70 °C, de préférence dans la plage de 50 à 65 °C, de préférence dans la plage de 50 à 60 °C.

15. Procédé selon l'une des revendications 1 à 14, **caractérisé en ce que** la température des noyaux à enrober dans le lit fluidisé se situe dans la plage de 20 à 55 °C, de préférence dans la plage de 40 à 55 °C et en particulier dans la plage de 45 à 50 °C.

16. Procédé selon l'une des revendications 1 à 15, **caractérisé en ce que** l'enrobage, après l'application de la totalité de la quantité à appliquer, est maintenu à la température d'application pendant 30 minutes à 120 minutes, de préférence pendant 60 minutes à 90 minutes.

17. Procédé selon l'une des revendications 1 à 16, **caractérisé en ce que** les rapports quantitatifs optimisés des composants qui sont nécessaires pour l'étape (ii) de la revendication 1, l'épaisseur de la couche à former ainsi que la température optimale et la durée optimale pour la formation du film cohérent, ou encore les paramètres pour la préparation des échantillons et pour le procédé de détermination des essais MVT, pour la détermination de la température minimale de pelliculage et du temps minimal de pelliculage, sont déterminés préalablement à l'application des matériaux.

## Figur 1

Abluft

Coating-
behälter

Weichmacherdüse

Weichmacher-
zuführungsschlauch

Pulverzuführung

Befilmungsgut

Zuluft

## Figur 2

Weichmacher-
zuführung

Alufolie

Pulverzuführung

Rotierender Zylinder

Figur 3

Figur 4

Proben unmittelbar nach Herstellung

Probe nach 10 min auf der MVT/MFT-
Bestimmungsplatte

Verfilmungsfront bei 60°C

Unverfilmte
Region

Verfilmte
Region

Probe nach 15 min auf der MVT/MFT-
Bestimmungsplatte

Verfilmungsfront bei 50°C

Unverfilmte Region

Verfilmter
Region

Probe nach 20 min auf der MVT/MFT-Bestimmungsplatte

Verfilmungsfront
bei 40°C

Unverfilmte Region

Verfilmte Region

Probe nach 150 min auf der MVT/MFT-Bestimmungsplatte

Verfilmungsfront
bei 40°C

Unverfilmte Region

Verfilmte Region

Figur 5

Freisetzung aus Diclofenac-Pellets solvent-free gecoatet mit 20% Poly(EA-MMA-TAMCl)-Pulver -Auftrag (1.Charge) nach 0 min Curing

Figur 6

**Freisetzung aus Diclofenac-Pellets solvent-free gecoatet mit 20% Poly(EA-MMA-TAMCl)-Pulver-Auftrag nach Curing über 70 min bei verschiedene Temperaturen**

EP 1 765 307 B1